# EUROPEAN PATENT SPECIFICATION

(11) **EP 3 875 077 B1**
(45) Date of publication and mention of the grant of the patent: **13.12.2023**
(21) Application number: 20160419.6
(22) Date of filing: 02.03.2020
(51) Int. Cl.: A61K 9/20, A61K 9/50, A61K 31/137, A61P 25/00, A61P 25/04

(54) **DOSAGE FORM PROVIDING PROLONGED RELEASE OF TAPENTADOL PHOSPHORIC ACID SALT**
DARREICHUNGSFORM MIT VERLÄNGERTER FREISETZUNG VON TAPENTADOLPHOSPHORSÄURESALZ
FORME POSOLOGIQUE À LIBÉRATION PROLONGÉE DE SEL D'ACIDE PHOSPHORIQUE DE TAPENTADOL

(43) Date of publication of application: 08.09.2021
(73) Proprietor: Grünenthal GmbH, 52078 Aachen (DE)
(72) Inventor: BERTRAM, Ulrike, 52066 Aachen (DE); REINHOLD, Ulrich, 52076 Aachen (DE); GROSSE, Christian, 52072 Aachen (DE); HARTMANN, Carmen, 50859 Köln (DE)
(74) Representative: Kutzenberger Wolff & Partner

(56) References cited:
- WO-A1-2012/051246
- WO-A1-2017/182438
- WO-A2-2008/051617
- US-A1- 2020 038 344
- Basf ET AL: "Kollidon SR - Technical Information BASF, September 2015.", BASF, 1 September 2015 (2015-09-01), pages 4-10, XP055972547, Retrieved from the Internet: URL:https://pharma.basf.com/technicalinfor mation/30071321/kollidon-sr [retrieved on 2022-10-18]
- Rowe C Raymond ET AL: "Handbook of pharmaceutical excipients, sixth edition", , 1 January 2009 (2009-01-01), page 122-126, 262-267; 286-288, 298-300, 326-329, 525-5, XP055972578, Retrieved from the Internet: URL:https://adiyugatama.files.wordpress.co m/2012/03/handbook-of-pharmaceutical-excip ients-6th-ed.pdf [retrieved on 2022-10-18]
- CHI L LI ET AL: "The use of hypromellose in oral drug delivery", JOURNAL OF PHARMACY AND PHARMACOLOGY : JPP, JOHN WILEY & SONS LTD, LONDON, GB, vol. 57, no. 5, 1 May 2005 (2005-05-01), pages 533-546, XP002693776, ISSN: 0022-3573, DOI: 10.1211/0022357055957 [retrieved on 2010-02-18]
- JAIN ARUN KUMAR ET AL: "The influence of hydroxypropyl methylcellulose (HPMC) molecular weight, concentration and effect of food onin vivoerosion behavior of HPMC matrix tablets", JOURNAL OF CONTROLLED RELEASE, ELSEVIER, AMSTERDAM, NL, vol. 187, 10 May 2014 (2014-05-10), pages 50-58, XP028860086, ISSN: 0168-3659, DOI: 10.1016/J.JCONREL.2014.04.058

## Description

The invention relates to a tablet providing prolonged release of Tapentadol, wherein Tapentadol is present as dihydrogenphosphate salt. The tablet has satisfactory mechanical properties, e.g. in terms of resistance to crushing and friability, and can be prepared under greatly facilitated tableting conditions, especially at reduced compressing force.

Tablets are formed by applying a compression force to a powder and consolidating that powder into a compact within a die of a press. Two processes occur simultaneously when the tablet is made: compaction and compression. By definition, compaction is the increase in mechanical strength of powder under force due to the consolidation of particles. Thus, compaction is related to particle consolidation and bonding, which has a direct effect on the tablet hardness and friability. Compression, on the other hand, is defined as a reduction in bulk volume of the powder under force due to displacement of air between particles. Compression results in a reduction of void space between solid particles, which means a decrease in porosity of a tablet (for details see e.g. Y. Qiu et al., Developing Solid Oral Dosage Forms, Pharmaceutical Theory & Practice, 2nd ed., Elsevier, 2017, pp. 940-942).

Tablet manufacture is influenced by a number of interdependent parameters including
- the desired properties of the tablet (e.g., mechanical strength, weight variability, disintegration time, size and shape),
- the nature and amount of the excipients of the solid material to be formed into tablets, and
- the conditions in the tableting machine (e.g., compression force, tableting speed, punch dwell time in the die, punch geometry, tooling material, etc.).

As far as the desired properties of the tablet are concerned, these highly depend upon pharmacological, i.e. pharmacokinetic and pharmacodynamic considerations. Potency of drug and intended administration frequency determine the dose of drug to be contained in a tablet. Release characteristics determine that nature and amount of excipients as well as the overall distribution of excipients within the tablet. For example, matrix retardation requires considerable amounts of prolonged release matrix material in which the drug is embedded. Sufficient mechanical strength is necessary in order to enable e.g. ejection of the compacts from the dies in the course of the tableting process and release of tablets from suitable packaging such as blisters.

As far as nature and amount of excipients are concerned, powder excipients are used as binders for cohesiveness, as fillers for tablet tensile strength, as lubricants for successful processing of a blend or granulation into a tablet, as disintegrants to facilitate the disintegration of the tablet *in vivo,* as controlled release materials for retarding dissolution, and sometimes as glidants to aid poorly flowing mixtures. Each excipient is predominantly either elastic, or plastic, or brittle. Elastic particles deform during compression up to their elastic limit after which they will fracture. Prior to reaching that stretch limit, the deformation of elastic materials is reversible. Elastic powders expand after compression forces are removed (relaxation). Plastic deformation maintains shape after compressive forces are removed, and therefore deforms without recovery to provide good particulate binding. A brittle component will fracture under compression stress when the force goes beyond its deformation limit. While more surface area forms after brittle fracture, it also results in more friction and resistance to movement within the die during compression. Plastic and brittle excipients form physical shapes and add stability to the shape after compression.

As far as the conditions in the tableting machine are concerned, modern sophisticated tableting machines all employ the same general concepts of operation: die fill, tablet compression, tablet ejection, and table scrape-off. The quality of the compact depends on the compression and consolidation of the powder mass, decompression of the compact, ejection from the die, and subsequent scrape-off from the lower punch. Because these viscoelastic properties are time dependent, both the magnitude and the rate of application (and release) of the compression force affect tablet quality.

Tablet punches are used to compress a blend or granulation into a compacted unit dose.

In roller compaction tableting machines (rotary tablet presses) first, the bottom punch is lowered so that the feeder can dispense powder into a die. A scraper removes excess powder so that the formulation is level with the top of the die. The amount of powder in the die is the fill. The upper punch is then lowered into the die. The pre-compression rolls apply pressure to deaerate the fill in the die by forcing the particles closer together. This is followed by the main compression rollers applying force to compress the powder into a compact. The upper punch is then lifted from the die by the upper-lifting cam. The lower punch is then raised by the ejection cam to eject the compact out of the die so that a scraper can remove it. Compression enables particle-to-particle bonding for the purpose of making a tablet of sufficient cohesive strength or hardness, to maintain its shape and appearance.

In excenter press tableting machines (excenter tablet presses), there is no pre-compression and the force is not transmitted by compression rollers. The overall principle of compression and compaction, however, is very similar.

When powder is added to a die, there is space between the particles. As the powder undergoes compression, the particles are shown to consolidate and the powder fill deaerates. The die constrains powder movement and provides time for cohesive bonding of particles to each other. This is followed by particle rearrangement, plastic and elastic deformation, and then fragmenting of the brittle components once they have exceeded their deformation limit. The pressure enables formation of interparticulate bonds that provide the compact's tensile strength. After ejection from the die, the compact undergoes a type of elastic recovery (relaxation), whereby the elastic materials partially return to their pre-compressed forms. The cohesiveness of compressed particles holds the shape of the compact after compression.

Some of the deformation characteristics are time-dependent and therefore, machine characteristics can have a major effect on the tableting performance. These characteristics determine the rate of force application, dwell time (i.e. the time of maximum compression force, which depends on the punch head flat diameter and the tangential velocity), and the rate of decompression. Typically for materials that undergo plastic deformation, as machine speed is increased there is less time for stress relaxation.

Tablets need to withstand the rigors of manufacturing, packaging, shipping, and distribution. According to FDA's Quality Attribute Considerations for Chewable Tablets, Guidance for Industry, August 2018, chewable tablets should have a hardness of < 12 kp (~19.6 N), whereas it is recognized that tablet size and shape are important in determining acceptable tablet hardness. For example, for a very small tablet, a hardness value of 20 N may be quite high. Further, it has been recommended that fast disintegrating tablets providing immediate drug release should have a hardness of from about 70 to 100 N, whereas tablets providing prolonged drug release should have a hardness of from about 100 to 200 N. The larger the tablets, the harder they need to be in order to withstand the attrition during manufacture, packaging, shipping, and distribution. Therefore, with regard to prolonged release tablets, tablet hardness should be at least 100 N, preferably at least 150 N.

Within certain limits, tablet hardness is a function of the compression force; the higher the compression force, the higher the tablet hardness. Thus, for a given excipient mixture, increased tablet hardness can often be achieved by increasing the compression force. However, this may simultaneously require a reduction of machine speed in order to properly adjust the dwell time thereby reducing the overall tablet yield per hour. This is because interparticulate and adhesive bonding can both be affected by not only the compression force but also by the punch dwell time during which the powder is compressed in the die. Dwell time refers to the length of time that a punch is compressing powder in the die. It is a function of machine speed, whereby faster tableting leaves less time for the punch to compress the powder in the die. Lengthening the dwell time provides more time for particle deformation, fragmentation for increased surface area, and for interparticulate bonds to form. When changing compression force it should be also borne in mind that this not only affects tablet hardness. Harder tablets may have longer disintegration times and dissolution rates. When finding the balance between compression force and dwell time, tablet attributes must be considered (for details see e.g. M.T. Ende et al., Chemical Engineering In The Pharmaceutical Industry, Drug Product Design, Development, and Modeling, 2nd ed., Wiley, 2019, pp. 228-232; J. Swarbrick, M. Bogda, Encylopedia of Pharmaceutical Technology, 3rd ed., Informa Healthcare, 2007, Tablet Compression: Machine Theory, design, and Process Troubleshooting, 3611-2629).

Therefore, increasing compression force in order to improve tablet hardness can require reduction of machine speed resulting in economic disadvantages due to reduced output.

Furthermore, compression forces cannot be increased *ad ultimo* due to material properties (tooling constraints). Tablet tooling strength is a function of tablet size, shape and fill weight. Several tablet shapes and sizes therefore allow for maximum compression forces of less than 10 kN. In consequence, tableting machines that are equipped with punches of such size and shape already operate at compression forces close to their maximum tooling force and under these conditions, there is little room for increasing compression force in order to increase tablet hardness (for details see e.g. L.L. Augsburger et al., Pharmaceutical Dosage Forms: Tablets, 3rd ed., informa healthcare, 2008, pp. 26-30; pp. J. Zheng, Formulation and Analytical Development for Low-Dose Oral Drug Products, Wiley 2009, 150-155).

Various tablets containing Tapentadol are known from the prior art.

WO 2003/035054 A1 relates to a pharmaceutical formulation which is characterized by delayed release of Tapentadol HCl in a matrix with delayed release of the active ingredient. Said matrix contains between 1 and 80 wt. % of at least one hydrophilic or hydrophobic polymer as a pharmaceutically acceptable matrix forming agent. The granules were pressed on a EKO eccentric press (Korsch) to 6 x 15 mm size oblong tablets with a breaking notch.

EP 2 942 054 A1 relates to a slow-release pharmaceutical formulation containing Tapentadol HCl in a slow release matrix, wherein the matrix contains between 15 and 50 wt.-% of mono-, di- and triglycerides of saturated fatty acids with a chain length between 16 and 22 carbon atoms or a mixture thereof. The influence of compression force and tablet dimensions on tablet hardness and dissolution behavior was investigated. At a constant force of 27 kN a tablet hardness of only 53 to 79 N could be achieved, while lower forces provided even poorer tablet hardness.

WO 2008/051617 A2 relates to a method for producing a dry granule composition comprising compressing a pharmaceutical composition to 800 to 900 kPa hardness to produce one or more slugs, and milling the one or more slugs with an oscillating granulator to form granules. An exemplary pharmaceutical formulation contains Tapentadol HCl, hypromellose, microcrystalline cellulose, colloidal silicon dioxide and magnesium stearate. Figure 7 to 9 show the influence of compression force on tablet hardness. At compression forces of 2000 to 3000 lbs (∼8.9 to ~13.3 kN), tablets having a hardness of about 7 to 20 kp (-69 to -196 N) were obtained.

WO 2015/014980 A1 discloses a pharmaceutical composition comprising (a) dissolved Tapentadol HCl, (b) organic solvent with a boiling point of 110° to 350° C, and (c) solid carrier. If a rotary press is applied for manufacture of the tablets, the main compression force can range from 1 to 50 kN, preferably 3 to 40 kN. The resulting tablets can have a hardness of 30 to 400 N, more preferred of 50 to 250 N, particularly preferably of 30 to 180 N, more preferably 40 to 150 N.

D.V. Reddy et al., Journal of Pharmacy Research 2014. 8(10), 1368-1374 reports about manufacture of extended release tablets of Tapentadol HCl using hydrophilic-hydrophobic polymer combinations manufactured by wet granulation.

S.M. Afzal et al., Int J A PS BMS 2017, 6(1), 1-21 relates to the preparation of Tapentadol HCl sustained release matrix tablets by sintering technique.

S.K. Paramasivan et al., GSC Biological and Pharmaceutical Sciences, 2018, 04(03), 042-048 relates to tablets comprising Tapentadol manufactured by direct compression in a rotary tablet compression machine.

The known tablets containing Tapentadol are not satisfactory in every respect, especially as compression of tablets from the starting materials requires considerable compression forces in order to achieve satisfactory mechanical strength, e.g. hardness.

It would be desirable to provide oral dosage forms of Tapentadol that are easy to manufacture and do not require e.g. thermoforming or multiple coating steps. The oral dosage forms should preferably be provided in form of tablets, preferably monolithic tablets, i.e. should not require preparation of a multitude of particles. The preparation of the oral dosage forms should be possible on standard equipment on industrial high throughput scale, preferably by compression of powder mixtures possibly involving granulation (dry granulation or wet granulation), preferably, however, by direct compression of powder mixtures.

There is a demand for formulations of Tapentadol that can be compressed into tablets by means of conventional tableting machines at high machine speeds providing satisfactory tablet properties, especially with respect to hardness, and that allow for a broad variation of tablet sizes and shapes without reaching the tooling strength limits of conventional tableting equipment.

It is an object of the invention to provide pharmaceutical dosage forms of Tapentadol that have advantages compared to the pharmaceutical dosage forms of the prior art.

This object has been achieved by the subject-matter of the patent claims.

### SUMMARY OF THE INVENTION

The invention provides a pharmaceutical dosage form according to claim 1. The pharmaceutical dosage form is a tablet and comprises Tapentadol for oral administration twice daily; wherein Tapentadol is present as a salt with phosphoric acid, which is a crystalline form and/or amorphous form of the dihydrogenphosphate salt of Tapentadol, a solvate, an ansolvate, and/or a polymorph thereof; wherein the dosage form provides prolonged release of Tapentadol; and wherein the weight equivalent dose of Tapentadol that is contained in the pharmaceutical dosage form is within the range of from 10 to 300 mg based on the free base of Tapentadol (Mᵣ = 221.3 g/mol).

It has been surprisingly found that salts of Tapentadol with phosphoric acid (H₃PO₄) are particularly useful for pharmaceutical dosage forms that provide prolonged release of Tapentadol. It has been surprisingly found that compared to conventional Tapentadol hydrochloride, the preparation of compressed tablets comprising salts of Tapentadol with phosphoric acid requires significantly reduced compression forces in order to achieve a desired target breaking strength of the tablets. This effect neither depends upon the particle size of the salt of Tapentadol with phosphoric acid, nor upon the polymorphic form of the salt of Tapentadol with phosphoric acid. Further, this effect is also observed with various different excipients, i.e. is attributable to the properties of the salt of Tapentadol with phosphoric acid as such.

Salts of Tapentadol with phosphoric acid are known, e.g. from WO 2010/096045, WO 2012/010316 A1, WO 2012/051246 A1 and WO 2017/182438 A1. However, none of these references addresses reduction of compressing forces in order to achieve a desired target breaking strength of the tablets.

Dosage forms providing prolonged release of Tapentadol are also known, e.g. from WO 03/035053 A1, WO 2006/002886 A1, and WO 2009/092601 A1. However, none of these references addresses reduction of compressing forces in order to achieve a desired target breaking strength of the tablets.

According to the instructions for use, the commercial Tapentadol tablets Palexia^{®} retard contain Tapentadol as hydrochloride salt, whereas the tablet core additionally contains hypromellose, microcrystalline cellulose, highly disperse silicon dioxide, and magnesium stearate. Thus, Palexia^{®} retard tablets contain a prolonged release matrix of hypromellose. The tablet cores are film coated with a composition comprising hypromellose, lactose monohydrate, talcum, macrogol 6000 and colorants. These tablets are in accordance with WO 03/035053 A1 and with the comparative examples contained herein in the experimental section.

### BRIEF DESCRIPTION OF THE DRAWINGS

Figure 1 shows the XRPD spectrum of Tapentadol dihydrogenphosphate (mixed form) as obtained according to Example 1.1.
Figure 2 shows the XRPD spectrum of Tapentadol dihydrogenphosphate hemihydrate as obtained according to Example 1.4.
Figure 3 shows the DSC plot of Tapentadol dihydrogenphosphate hemihydrate as obtained according to Example 1.4.
Figure 4 shows the XRPD spectrum of Tapentadol dihydrogenphosphate anhydrate as obtained according to Example 1.6.
Figure 5 shows the DSC plot of Tapentadol dihydrogenphosphate anhydrate as obtained according to Example 1.6.
Figure 6 shows three photographs of different particles size of the salt of Tapentadol with phosphoric acid (Figure 6A shows relatively fine particles, Figure 6B relatively coarse particles) and of the salt of Tapentadol with hydrochloric acid (Figure 6C).

### DETAILED DESCRIPTION OF THE INVENTION

A first aspect of the invention relates to a pharmaceutical dosage form according to claim 1. The pharmaceutical dosage form is a tablet and comprises Tapentadol for oral administration twice daily; wherein Tapentadol is present as a salt with phosphoric acid, which is a crystalline form and/or amorphous form of the dihydrogenphosphate salt of Tapentadol, a solvate, an ansolvate, and/or a polymorph thereof; wherein the dosage form provides prolonged release of Tapentadol; and wherein the weight equivalent dose of Tapentadol that is contained in the pharmaceutical dosage form is within the range of from 10 to 300 mg based on the free base of Tapentadol.

Tapentadol, i.e. (-)-(1R,2R)-3-(3-dimethylamino-1-ethyl-2-methyl-propyl)-phenol, is a synthetic, centrally acting analgesic which is effective in the treatment of moderate to severe, acute or chronic pain. The synthesis of the free base of Tapentadol is known e.g. from EP-A 693 475.

The pharmaceutical dosage form according to the invention contains Tapentadol as a salt with phosphoric acid, which is a crystalline form and/or amorphous form of the dihydrogenphosphate salt of Tapentadol, a solvate, an ansolvate, and/or a polymorph thereof. While it is contemplated that the pharmaceutical dosage form according to the invention may contain mixtures of different salts of Tapentadol or mixtures of salt(s) with the free base of Tapentadol (e.g. the non-salt form of Tapentadol), preferably the total amount of Tapentadol that is contained in the pharmaceutical dosage form is present as a salt with phosphoric acid.

The salt of Tapentadol with phosphoric acid is the dihydrogenphosphate salt of Tapentadol, which may optionally be solvated (e.g. hydrated) or ansolvated (e.g. anhydrated). Preferably, the dihydrogenphosphate salt of Tapentadol is selected from the group consisting of ansolvated dihydrogenphosphate salt of Tapentadol, anhydrated dihydrogenphosphate salt of Tapentadol, solvated dihydrogenphosphate salt of Tapentadol, hydrated dihydrogenphosphate salt of Tapentadol, and any mixtures of the foregoing. The salt of Tapentadol may be present in form of a single polymorph or as a mixture of different polymorphs in any mixing ratio.

In a preferred embodiment, the salt of Tapentadol with phosphoric acid is the dihydrogenphosphate salt which is present in essentially pure crystalline form of Tapentadol dihydrogenphosphate hemihydrate salt.

In a preferred embodiment, the salt of Tapentadol with phosphoric acid is the dihydrogenphosphate salt which is present in essentially pure crystalline form of Tapentadol dihydrogenphosphate anhydrate salt.

In a preferred embodiment, the salt of Tapentadol with phosphoric acid is a crystalline dihydrogenphosphate salt having characteristic X-ray powder diffraction peaks at 5.1 , 14.4, 17.7, 18.3 and 21.0 degrees 2Θ (± 0.2 degrees 2Θ); preferably being characterized by one or more further XRPD diffraction peak(s) at 8.7, 17.6, 20.3, 22.1 and/or 23.4 degrees 2Θ (± 0.2 degrees 2 Θ). Alternatively, the crystalline dihydrogenphosphate salt can be characterized by the XRPD diffraction peak(s) at degrees 2Θ ± 0.2 degrees 2Θ (intensity %): 5.1 (100), 8.7 (10), 10.1 (1), 12.5 (3), 13.4 (4), 14.4 (37), 15.3 (4), 17.6 (12), 17.7 (15), 18.3 (27), 19.1 (7), 20.3 (1 1), 21.0 (25), 21.6 (9), 22.1 (18), 23.4 (14), 24.8 (7), 25.0 (17), 25.3 (12), 26.0 (9), 26.5 (7), 26.8 (9), 28.0 (5), 28.4 (4), 28.9 (10), 29.2 (6), 29.4 (4), and 29.6 (2). Alternatively, the crystalline dihydrogenphosphate salt can be characterized by the XRPD diffraction peak(s) at degrees 2Θ ± 0.2 degrees 2Θ: 5.1 , 8.7, 10.1 , 12.5, 13.4, 14.4, 15.3, 17.6, 17.7, 18.3, 19.1 , 20.3, 21.0, 21.6, 22.1 , 23.4, 24.8, 25.0 and 25.3.

In another preferred embodiment, the salt of Tapentadol with phosphoric acid is a crystalline dihydrogenphosphate salt having characteristic X-ray powder diffraction peaks at 5.1 , 14.3, 17.6, 18.5 and 21.1 degrees 2Θ (± 0.2 degrees 2Θ); preferably being characterized by one or more further XRPD diffraction peak(s) at 8.9, 20.5, 22.4, 23.5 and/or 24.3 degrees 2Θ (± 0.2 degrees 2Θ). Alternatively, the crystalline dihydrogenphosphate salt can be characterized by the XRPD diffraction peak(s) at degrees 2Θ ± 0.2 degrees 2Θ (intensity %): 5.1 (100), 8.9 (3), 12.4 (5), 13.5 (4), 14.3 (42), 15.2 (4), 17.6 (21), 18.5 (18), 19.1 (7), 20.5 (14), 21.1 (28), 21.7 (9), 22.4 (14), 23.5 (15), 24.3 (6), 24.9 (19), 25.1 (16), 25.8 (3), 26.2 (12), 26.4 (13), 26.7 (5), 27.3 (2), 28.2 (5), 28.8 (8), 29.1 (1 1), 29.4 (5), and 29.6 (5). Alternatively, the crystalline dihydrogenphosphate salt can be characterized by the XRPD diffraction peak(s) at degrees 2Θ ± 0.2 degrees 2Θ: 5.1 , 8.9, 12.4, 13.5, 14.3, 15.2, 17.6, 18.5, 19.1 , 20.5, 21.1 , 21.7, 22.4, 23.5, 24.3, 24.9, and 25.1.

Preferably, the dihydrogenphosphate salt of Tapentadol has an average particle size, expressed as surface mean D[3,2] (Sauter Mean Diameter) and determined by laser diffraction in the dry mode in accordance with ISO 13320:2020, within the range of 5.0 to 500 µm, preferably 50±40 µm, or 75±40 µm, or 100±40 µm, or 125±40 µm, or 150±40 µm, or 175±40 µm, or 200±40 µm, or 225±40 µm, or 250±40 µm, or 275±40 µm, or 300±40 µm, or 325±40 µm, or 350±40 µm, or 375±40 µm, or 400±40 µm, or 425±40 µm, or 450±40 µm.

In a preferred embodiment, the dihydrogenphosphate salt of Tapentadol is present in crystalline form. In another preferred embodiment, the salt of Tapentadol with phosphoric acid is present in amorphous form. It is further contemplated that the pharmaceutical dosage form may contain mixtures of crystalline salt of Tapentadol with phosphoric acid with amorphous salt of Tapentadol with phosphoric acid in any mixing ratio. Preferably, essentially the total amount of the salt of Tapentadol with phosphoric acid that is present in the pharmaceutical dosage form is crystalline.

For the purpose of the specification, the dihydrogenphosphate salt of Tapentadol is to be regarded as the acid addition salt of 1 mole orthophosphoric acid to 1 mole of Tapentadol. It is believed that one proton of orthophosphoric acid (H₃PO₄) protonates the amino group of Tapentadol thereby forming an ammonium ion, whereas the remainder of orthophosphoric acid, i.e. the dihydrogenphosphate anion (H₂PO₄⁻), forms the counterion:

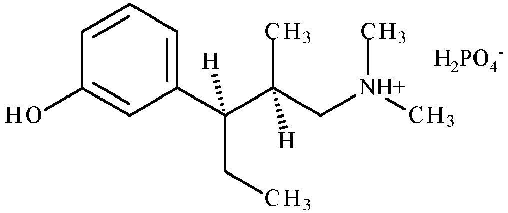

For the purpose of the specification, reference to "Tapentadol" shall include the salt of Tapentadol with phosphoric acid, which is a crystalline form and/or amorphous form of the dihydrogenphosphate salt of Tapentadol, a solvate, an ansolvate, and/or a polymorph thereof.

The dihydrogenphosphate salt of Tapentadol, may be present in form of any solvate, e.g. hydrate, ansolvate, e.g. anhydrate, and polymorphic form, e.g. any crystalline form and/or amorphous form. With regard to these forms and the preparation thereof reference is made to WO 2012/010316 A1 and WO 2017/182438 A1.

The weight equivalent dose of Tapentadol that is contained in the pharmaceutical dosage form according to the invention is within the range of from 10 to 300 mg based on the free base of Tapentadol, e.g. 25 mg, 50 mg, 100 mg, 150 mg, 200 mg, or 250 mg. The free base of Tapentadol (i.e. the free molecule) has a molecular weight of 221.3 g/mol. Thus, 10 mg of the free base of Tapentadol correspond to 0.0452 mmol, whereas 300 mg of the free base of Tapentadol correspond to 1.3556 mmol. Thus, in other words, the pharmaceutical dosage form contains a mole equivalent dose of Tapentadol within the range of from 0.0452 to 1.3556 mmol.

The hydrochloride salt of Tapentadol (anhydrate) has a molecular weight of 257.8 g/mol, whereas the dihydrogenphosphate salt of Tapentadol (anhydrate) has a molecular weight of 319.3 g/mol. Thus, when the pharmaceutical dosage form according to the invention contains e.g. a weight equivalent dose of 100 mg based on the free base of Tapentadol (0.4518 mmol), it actually contains 144.3 mg dihydrogenphosphate salt of Tapentadol (0.4518 mmol).

Unless expressly stated otherwise, dose information for Tapentadol is thus expressed as equivalent weight relative to the free base of Tapentadol, i.e. the non-salt form, the non-solvate form, the non-cocrystal form, and the non-aggregate form of Tapentadol with any other molecules. It is contemplated that the form of Tapentadol that is actually contained in the pharmaceutical dosage form, i.e. the dihydrogenphosphate salt of Tapentadol, may be present in form of any polymorph and/or solvate and/or cocrystal and/or any other aggregate of such salt with other molecules.

Unless expressly stated otherwise, all percentages are weight percent and are related to the total weight of the pharmaceutical dosage form. When the pharmaceutical dosage form is coated, the weight of the coating is included in the total weight of the pharmaceutical dosage form.

While it is contemplated that besides Tapentadol the pharmaceutical dosage form according to the invention may contain additional pharmacologically active ingredients, Tapentadol is preferably the sole pharmacologically active ingredient that is contained in the pharmaceutical dosage form.

Preferably, Tapentadol is homogeneously distributed over the pharmaceutical dosage form according to the invention.

The pharmaceutical dosage form according to the invention is devoted for oral administration, preferably by swallowing the pharmaceutical dosage form as a whole. Thus, the pharmaceutical dosage form according to the invention is preferably not devoted for buccal or sublingual administration where the pharmaceutical dosage form would be devoted to remain within the oral cavity.

The pharmaceutical dosage form according to the invention is devoted for administration twice daily. Thus, the pharmaceutical dosage form according to the invention contains 50% of the daily dose of Tapentadol that is intended for administration in order to bring about the desired therapeutic effect.

Administration twice daily may proceed at intervals of about every 12 hours, although such regimen is not to be strictly followed. For the purpose of the specification, twice daily shall also encompass any administration regimen where two pharmaceutical dosage forms according to the invention are administered during a period of about 24 hours, where the two administrations must be separated from one another by at least 4 hours, preferably at least 8 hours.

The pharmaceutical dosage form according to the invention provides prolonged release of Tapentadol. For the purpose of the specification, prolonged release means not immediate release. Prolonged release includes controlled release, delayed release, extended release, staggered release, repeat action release, sustained release and evenly sustained release. Prolonged release preferably means a release with a reduced release rate, to obtain a therapeutic effect upright, to reduce toxic effects or for some other therapeutic purpose.

Prolonged release may be based upon different technologies that are known to the skilled person. According to the invention, prolonged release is based upon a prolonged release matrix comprising as prolonged release matrix material hydroxypropylmethylcellulose in which Tapentadol is embedded.

Preferably, the pharmaceutical dosage form according to the invention after oral administration provides plasma levels of Tapentadol providing pain relief (analgesia) for a duration of at least 6 hours, preferably at least 8 hours, more preferably at least 10 hours, most preferably at least 12 hours.

Preferably, the pharmaceutical dosage form according to the invention provides an *in vitro* dissolution profile, when measured by the USP Paddle Method at 50 rpm in 900 ml aqueous phosphate buffer at pH 6.8 at 37° C, wherein
- after 0.5 hour 20±20 wt.-%; preferably 20±15 wt.-%; more preferably 20±10 wt.-%;
- after 4 hours 60±20 wt.-%; preferably 60±15 wt.-%; more preferably 60±10 wt.-%; and
- after 12 hours at least 60 wt.-%; preferably at least 70 wt.-%; more preferably at least 80 wt.-%
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml aqueous phosphate buffer at pH 6.8 at 37° C, wherein
- after 1 hour 25±15 wt.-%; preferably 25±10 wt.-%; more preferably 25±5.0 wt.-%;
- after 2 hours 35±20 wt.-%; preferably 35±10 wt.-%; more preferably 35±5.0 wt.-%;
- after 4 hours 50±20 wt.-%; preferably 50±10 wt.-%; more preferably 50±5.0 wt.-%;
- after 8 hours 80±20 wt.-%; preferably 80±10 wt.-%; more preferably 80±5.0 wt.-%;
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml aqueous buffer at pH 4.5 at 37° C, wherein
- after 0.5 hour 20±20 wt.-%; preferably 20±15 wt.-%; more preferably 20±10 wt.-%;
- after 4 hours 60±20 wt.-%; preferably 60±15 wt.-%; more preferably 60±10 wt.-%; and
- after 12 hours at least 60 wt.-%; preferably at least 70 wt.-%; more preferably at least 80 wt.-%
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml aqueous buffer at pH 4.5 at 37° C, wherein
- after 1 hour 25±15 wt.-%; preferably 25±10 wt.-%; more preferably 25±5.0 wt.-%;
- after 2 hours 35±20 wt.-%; preferably 35±10 wt.-%; more preferably 35±5.0 wt.-%;
- after 4 hours 50±20 wt.-%; preferably 50±10 wt.-%; more preferably 50±5.0 wt.-%;
- after 8 hours 80±20 wt.-%; preferably 80±10 wt.-%; more preferably 80±5.0 wt.-%;
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml 0.1 N HCl at pH 1.0 and 37 °C, wherein
- after 0.5 hour 20±20 wt.-%; preferably 20±15 wt.-%; more preferably 20±10 wt.-%;
- after 4 hours 60±20 wt.-%; preferably 60±15 wt.-%; more preferably 60±10 wt.-%; and
- after 12 hours at least 60 wt.-%; preferably at least 70 wt.-%; more preferably at least 80 wt.-%
of the Tapentadol that was originally contained in the dosage form have been released.

Preferably, the pharmaceutical dosage form according to the invention provides an *in vitro* dissolution profile, when measured by the USP Paddle Method at 50 rpm in 900 ml 0.1 N HCl at pH 1.0 and 37 °C, wherein
- after 1 hour 25±10 wt.-%; preferably 25±10 wt.-%; more preferably 25±5.0 wt.-%;
- after 2 hours 40±30 wt.-%; preferably 40±10 wt.-%; more preferably 40±5.0 wt.-%;
- after 4 hours 60±20 wt.-%; preferably 60±10 wt.-%; more preferably 60±5.0 wt.-%;
- after 8 hours 80±20 wt.-%; preferably 80±10 wt.-%; more preferably 80±5.0 wt.-%;
of the Tapentadol that was originally contained in the dosage form have been released.

Preferably, the pharmaceutical dosage form according to the invention provides resistance against ethanol induced dose dumping.

Preferably, the pharmaceutical dosage form according to the invention provides slower *in vitro* dissolution of Tapentadol in aqueous medium containing ethanol than in aqueous medium not containing ethanol.

Preferably, the pharmaceutical dosage form according to the invention provides slower dissolution of Tapentadol in 0.1 N HCl containing 5.0 vol.-% of ethanol (pH 1.0) than in 0.1 N HCl not containing 5.0 vol.-% of ethanol (pH 1.0), in each case when measured by the USP Paddle Method at 50 rpm in 900 ml at 37° C.

Preferably, the pharmaceutical dosage form according to the invention provides slower dissolution of Tapentadol in 0.1 N HCl containing 20 vol.-% of ethanol (pH 1.0) than in 0.1 N HCl not containing 20 vol.-% of ethanol (pH 1.0), in each case when measured by the USP Paddle Method at 50 rpm in 900 ml at 37° C.

Preferably, the pharmaceutical dosage form according to the invention provides slower dissolution of Tapentadol in 0.1 N HCl containing 40 vol.-% of ethanol (pH 1.0) than in 0.1 N HCL not containing 40 vol.-% of ethanol (pH 1.0), in each case when measured by the USP Paddle Method at 50 rpm in 900 ml at 37° C.

In preferred embodiments of the pharmaceutical dosage form according to the invention, the weight equivalent dose of Tapentadol that is contained in the pharmaceutical dosage form amounts to 25 mg, 50 mg, or 100 mg, in each case based on the free base of Tapentadol. Preferably, the pharmaceutical dosage form according to the invention has a total weight within the range of from 150 to 750 mg.

In preferred embodiments of the pharmaceutical dosage form according to the invention, the weight equivalent dose of Tapentadol that is contained in the pharmaceutical dosage form amounts to 150 mg, 200 mg or 250 mg, in each case based on the free base of Tapentadol. Preferably, the pharmaceutical dosage form according to the invention has a total weight within the range of from 300 to 1200 mg.

Preferably, the pharmaceutical dosage form according to the invention comprises one, two or more physiologically acceptable excipients.

Pharmaceutical excipients are known to the skilled person (cf. e.g. R. C. Rowe et al., Handbook of Pharmaceutical Excipients, Pharmaceutical Press; 6th edition 2009; E.-M. Hoepfner et al., Fiedler-Encyclopedia of Excipients, Editio Cantor, 6th edition 2008). For the purpose of the specification, a "pharmaceutical excipient" is preferably to be regarded as any pharmacologically inactive substance typically used as a carrier for the active ingredients of a medication. The pharmaceutical excipient may have a physiological effect, e.g. like a vitamin, but not a pharmacological effect, like a drug. Typical examples of pharmaceutical excipients include antiadherents, binders, coating materials, disintegrants, fillers, diluents, flavors, colorants, glidants, lubricants, preservatives, sorbents, surfactants, sweeteners, dyes, pigments, and the like.

Any of the foregoing excipients can be divided into sub-groups. For example, preservatives can be divided into antioxidants, buffers, antimicrobial substances and the like; whereas binders can be divided into solution binders and dry binders. Several excipients simultaneously exhibit different properties so that they can serve different purposes. For example, polyethylene glycol can be used as binder, plasticizer and the like.

Preferably, the pharmaceutical dosage form according to the invention, preferably the prolonged release matrix, comprises a binder.

Preferably, the binder is selected from the group consisting of cellulose, magnesium-aluminum silicates (e.g. bentonite), mono-, oligo- and polysaccharides (e.g. dextrose, lactose, mannose), sugar alcohols (e.g. lactitol, mannitol), starches (e.g. pregelatinized starch, hydrolyzed starch, modified starch), calcium phosphate, polyvinylpyrrolidone, and vinylpyrrolidone/vinyl acetate copolymers; preferably microcrystalline cellulose; more preferably silicified microcrystalline cellulose.

Preferably, the weight content of the binder is at least 5.0 wt.-%, more preferably at least 10 wt.-%, still more preferably at least 15 wt.-%, yet more preferably at least 20 wt.-%, even more preferably at least 25 wt.-%, most preferably at least 30 wt.-%, and in particular at least 35 wt.-%, in each case relative to the total weight of the pharmaceutical dosage form.

Preferably, the weight content of the binder is at most 85 wt.-%, more preferably at most 82.5 wt.-%, still more preferably at most 80 wt.-%, yet more preferably at most 77.5 wt.-%, even more preferably at most 75 wt.-%, most preferably at most 72.5 wt.-%, and in particular at most 70 wt.-%, in each case relative to the total weight of the pharmaceutical dosage form.

Preferably, the weight content of the binder is within the range of from 52±30 wt.-%, more preferably 52±27.5 wt.-%, still more preferably 52±25 wt.-%, yet more preferably 52±22.5 wt.-%, even more preferably 52±20 wt.-%, most preferably 52±17.5 wt.-%, and in particular 52±15 wt.-%, in each case relative to the total weight of the pharmaceutical dosage form.

When the pharmaceutical dosage form contains more than one binder, the above percentages refer to the total content of all binders that are contained in the pharmaceutical dosage form.

Preferably, the pharmaceutical dosage form according to the invention comprises a lubricant.

Preferably, the lubricant is selected from the group consisting of salts of fatty acids (e.g. magnesium stearate, calcium stearate, zinc stearate), fatty acids (e.g. stearic acid, palmitic acid), glyceryl fatty acid esters (e.g. glyceryl monostearate, glyceryl monobehenate, glyceryl dibehenate, glyceryl tribehenate), sorbitan monostearate, sucrose monopalmitate, sodium stearyl fumarate, hydrated magnesium silicate, and talc; preferably magnesium stearate.

Preferably, the weight content of the lubricant is at least 0.20 wt.-%, more preferably at least 0.25 wt.-%, still more preferably at least 0.30 wt.-%, yet more preferably at least 0.35 wt.-%, even more preferably at least 0.40 wt.-%, most preferably at least 0.45 wt.-%, and in particular at least 0.50 wt.-%, in each case relative to the total weight of the pharmaceutical dosage form.

Preferably, the weight content of the lubricant is at most 3.0 wt.-%, more preferably at most 2.8 wt.-%, still more preferably at most 2.6 wt.-%, yet more preferably at most 2.40 wt.-%, even more preferably at most 2.20 wt.-%, most preferably at most 2.00 wt.-%, and in particular at most 1.80 wt.-%, in each case relative to the total weight of the pharmaceutical dosage form.

Preferably, the weight content of the lubricant is within the range of from 0.1 to 1.0 wt.-%, more preferably 0.50±0.45 wt.-%, still more preferably 0.50±0.40 wt.-%, yet more preferably 0.50±0.35 wt.-%, even more preferably 0.50±0.30 wt.-%, most preferably 0.50±0.25 wt.-%, and in particular 0.50±0.20 wt.-%, in each case relative to the total weight of the pharmaceutical dosage form.

In the pharmaceutical dosage form according to the invention, Tapentadol is embedded in a prolonged release matrix.

The prolonged release matrix contains at least one physiologically acceptable polymer, which is hydroxypropylmethylcellulose, that serves the purpose of retarding release of the pharmacologically active ingredient from the pharmaceutical dosage form. Thus, said at least one physiologically acceptable polymer, which is hydroxypropylmethylcellulose, is part of the prolonged release matrix of the pharmaceutical dosage form according to the invention.

The prolonged release matrix comprises or essentially consists of a prolonged release matrix material selected from hydroxypropylmethylcellulose (HPMC). Preferably, the content of the prolonged release matrix material is within the range of 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, or 65±10 wt.-%, or 70±10 wt.-%, or 75±10 wt.-%, or 80±10 wt.-%, in each case relative to the total weight of the dosage form.

Preferably, the pharmaceutical dosage forms according to the invention comprise neither poly(alkylene oxide), e.g. polyethylene oxide), nor ethylene vinylacetate copolymers (EVA). For the purpose of the specification, poly(alkylene oxide) is distinguished from poly(alkylene glycol) by its molecular weight; polymers having a weight average molecular weight M_{w} of less than 100,000 g/mol are to be regarded as poly(alkylene glycol), whereas polymers having a weight average molecular weight M_{w} of 100,000 g/mol or more are to be regarded as poly(alkylene oxide).

Preferably, the hydroxypropylmethylcellulose is selected from hypromellose type 1828, 2208, 2906 and 2910 according to USP having the following methoxyl content hydroxypropoxyl content:

| | methoxyl (%) | | hydroxypropoxyl (%) | |
|---|---|---|---|---|
| type | min | max | min | max |
| 1828 | 16.5 | 20.0 | 23.0 | 32.0 |
| 2208 | 19.0 | 24.0 | 4.0 | 12.0 |
| 2906 | 27.0 | 30.0 | 4.0 | 7.5 |
| 2910 | 28.0 | 30.0 | 7.0 | 12.0 |

Preferably, the viscosity of the hydroxypropylmethylcellulose is within the range of 100,000±80,000 mPa s, more preferably 100,000±60,000 mPa s, still more preferably 100,000±40,000 mPa s, yet more preferably 100,000±20,000 mPa s.

Preferably, the number average molecular weight Mₙ of the hydroxypropylmethylcellulose is not more than 220,000 g/mol, more preferably not more than 180,000 g/mol, still more preferably not more than 140,000 g/mol, yet more preferably not more than 120,000 g/mol, even more preferably not more than 110,000 g/mol, most preferably not more than 86,000 g/mol, and in particular not more than 63,000 g/mol.

Preferably, the weight content of the physiologically acceptable polymer is at least 2.0 wt.-%, more preferably at least 3.0 wt.-%, still more preferably at least 4.0 wt.-%, yet more preferably at least 5.0 wt.-%, even more preferably at least 6.0 wt.-%, most preferably at least 7.0 wt.-%, and in particular at least 8.0 wt.-%, in each case relative to the total weight of the pharmaceutical dosage form.

Preferably, the weight content of the physiologically acceptable polymer is at most 62.5 wt.-%, more preferably at most 60 wt.-%, still more preferably at most 57.5 wt.-%, yet more preferably at most 55 wt.-%, even more preferably at most 52.5 wt.-%, most preferably at most 50 wt.-%, and in particular at most 47.5 wt.-%, in each case relative to the total weight of the pharmaceutical dosage form.

Preferably, the weight content of the physiologically acceptable polymer is within the range of from 30±28 wt.-%, more preferably 30±26 wt.-%, still more preferably 30±24 wt.-%, yet more preferably 30±22 wt.-%, even more preferably 30±20 wt.-%, most preferably 30±18 wt.-%, and in particular 30±16 wt.-%, in each case relative to the total weight of the pharmaceutical dosage form.

When the pharmaceutical dosage form contains more than one physiologically acceptable polymer, i.e. more than hydroxypropylmethylcellulose, serving the purpose of significantly retarding release of the pharmacologically active ingredient from the pharmaceutical dosage form, preferably cellulose ether, the above percentages refer to the total content of all such physiologically acceptable polymers, preferably cellulose ethers, that are contained in the pharmaceutical dosage form.

The pharmaceutical dosage form according to the invention comprises a weight equivalent dose of Tapentadol within the range of from 10 to 300 mg, e.g. 25 mg, 50 mg, 100 mg, 150 mg, 200 mg, or 250 mg, in each case based on the free base of Tapentadol, and a prolonged release matrix in which the dihydrogenphosphate salt of Tapentadol is embedded; wherein the prolonged release matrix comprises as prolonged release matrix material hydroxypropylmethylcellulose (HPMC) preferably in an amount of from 5.0 to 60 wt.-%, e.g. 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, in each case relative to the total weight of the dosage form.

In addition to the above ingredients, a prolonged release matrix may also contain suitable quantities of other materials, e.g. diluents, lubricants, binders, granulating aids, colorants, flavors and glidants that are conventional in the pharmaceutical art.

Pharmaceutical dosage forms containing prolonged release matrices in which Tapentadol is embedded may be prepared by conventional techniques that are known to the skilled person such as blending and direct compression, dry granulation, wet granulation, extrusion, and the like.

The pharmaceutical dosage form according to the invention is a tablet. Preferably, the tablet is monolithic. Monolithic tablets according to the invention mean tablets that are optionally film coated, wherein the core of the tablets contains a compressed powder and/or granulate mixture. In particular (i), tablets prepared by direct compression of powder mixtures, (ii) tablets prepared by compression of mixtures comprising granules obtained by dry granulation and optionally extragranular excipients, (iii) tablets prepared by compression of mixtures comprising granules obtained by wet granulation and optionally extragranular excipients, and (iv) tablets prepared by compression of mixtures comprising granules obtained by extrusion granulation and optionally extragranular excipients are all to be regarded as monolithic tablets in accordance with the invention. However, multiple unit pellet systems or other dosage forms where a multitude of particles of specific design, weight and shape is mixed with an outer matrix material and subsequently compressed into tablets where the outer matrix material forms a continuous phase in which the pellets or particles are embedded, are preferably not to be regarded as monolithic tablets. Of course, capsules filled with a multitude of loose particles are not to be regarded as monolithic either.

Preferably, the tablet has a breaking strength of at least 100 N, preferably at least 150 N, more preferably at least 200 N. The breaking strength is preferably determined in accordance with Ph. Eur. 10, chapter 2.9.8. *"Resistance to Crushing of Tablets".*

Another aspect of the invention related to a method for preparing a pharmaceutical dosage form according to the invention as described above.

In a preferred embodiment the method for the preparation according to the invention comprises the steps of
(a) providing a mixture containing essentially the total amount of Tapentadol (including the salt with phosphoric acid, which is a crystalline form and/or amorphous form of the dihydrogenphosphate salt of Tapentadol, a solvate, an ansolvate, and/or a polymorph thereof) to be contained in the dosage form and at least one prolonged release matrix material, which is hydroxypropylmethylcellulose, optionally together with one or more excipients;
(b) optionally granulating the mixture provided in step (a) thereby obtaining a granulate, wherein granulating preferably involves: (i) wet granulating by means of a solvent, preferably selected from water, ethanol, acetone, and any mixture thereof, optionally followed by drying; (ii) dry granulation; or (iii) extrusion;
(c) optionally mixing the granulate obtained in step (b) with one or more excipients thereby obtaining a granulate mixture;
(d) compressing the mixture provided in step (a) or the granulate obtained in step (b) or the granulate mixture obtained in step (c) into tablets;
(e) optionally, film coating the tablets compressed in step (d).

Preferably, compressing in step (d) of the process according to the invention is performed at a compression force of not more than 20 kN, more preferably not more than 15 kN, still more preferably not more than 10 kN, yet more preferably not more than 9.5 kN, even more preferably not more than 9.0 kN, most preferably not more than 8.75 kN, and in particular not more than 8.5 kN.

Preferably, compressing in step (d) of the process according to the invention is performed under conditions such that the compressed tablet has a breaking strength of at least 100 N, more preferably at least 150 N, still more preferably at least 200 N.

Preferably, the pharmaceutical dosage form according to the invention is not prepared by thermoforming such as hot-melt extrusion.

Preferably, the pharmaceutical dosage form according to the invention does not contain a multitude of particles or pellets of specific design, shape and weight that are optionally compressed into tablets wherein said particles or pellets form a discontinuous phase within a continuous phase of an outer matrix material.

Preferably, the pharmaceutical dosage form according to the invention does not contain an opioid antagonist. Opioid antagonists are entities that modify the response of opioid receptors. Opioid antagonists include Naloxone, Naltrexone, Diprenorphine, Etorphine, Dihydroetorphine, Nalinefene, Cyclazacine, Levallorphan, pharmaceutically acceptable salts thereof and mixtures thereof.

Another aspect of the invention relates to the pharmaceutical dosage form according to the invention as described above for use in the treatment of pain, wherein the dosage form is orally administered, preferably twice daily.

Preferably, the pain is chronic pain.

In preferred embodiments, the pharmaceutical dosage form according to the invention provides in a patient population of at least 10 patients, preferably at least 50 patients, an average value of Tₘₐₓ within the range of 5.0±3.0 hours after oral administration.

In preferred embodiments of the pharmaceutical dosage form according to the invention, the weight equivalent dose of Tapentadol that is contained in the pharmaceutical dosage form amounts to 50 mg based on the free base of Tapentadol and wherein the dosage form after oral administration in a patient population of at least 50 patients provides an average value of
- Cₘₐₓ within the range of 12±3 ng/ml; and/or
- AUCₗₐₛₜ within the range of 204±50 ng·h/ml; and/or
- AUC_{∞} within the range of 214±50 ng·h/ml.

In preferred embodiments of the pharmaceutical dosage form according to the invention, the weight equivalent dose of Tapentadol that is contained in the pharmaceutical dosage form amounts to 100 mg based on the free base of Tapentadol and wherein the dosage form after oral administration in a patient population of at least 50 patients provides an average value of
- Cₘₐₓ within the range of 29±6 ng/ml; and/or
- AUCₗₐₛₜ within the range of 440±100 ng h/ml; and/or
- AUC_{∞} within the range of 447±100 ng h/ml.

In preferred embodiments of the pharmaceutical dosage form according to the invention, the weight equivalent dose of Tapentadol that is contained in the pharmaceutical dosage form amounts to 150 mg based on the free base of Tapentadol and wherein the dosage form after oral administration in a patient population of at least 50 patients provides an average value of
- Cₘₐₓ within the range of 47±9 ng/ml; and/or
- AUCₗₐₛₜ within the range of 662±150 ng·h/ml; and/or
- AUC_{∞} within the range of 665±150 ng·h/ml.

In preferred embodiments of the pharmaceutical dosage form according to the invention, the weight equivalent dose of Tapentadol that is contained in the pharmaceutical dosage form amounts to 200 mg based on the free base of Tapentadol and wherein the dosage form after oral administration in a patient population of at least 50 patients provides an average value of
- Cₘₐₓ within the range of 64±12 ng/ml; and/or
- AUCₗₐₛₜ within the range of 890±200 ng·h/ml; and/or
- AUC_{∞} within the range of 895±200 ng·h/ml.

In preferred embodiments of the pharmaceutical dosage form according to the invention, the weight equivalent dose of Tapentadol that is contained in the pharmaceutical dosage form amounts to 250 mg based on the free base of Tapentadol and wherein the dosage form after oral administration in a patient population of at least 50 patients provides an average value of
- Cₘₐₓ within the range of 85±15 ng/ml; and/or
- AUCₗₐₛₜ within the range of 1141±250 ng h/ml; and/or
- AUC_{∞} within the range of 1145±250 ng h/ml.

### EXAMPLES

The following examples further illustrate the invention but are not to be construed as limiting its scope.

Commercial Tapentadol tablets Palexia^{®} retard contain Tapentadol as hydrochloride salt, whereas the tablet core additionally contains hypromellose, microcrystalline cellulose, highly disperse silicon dioxide, and magnesium stearate. Thus, Palexia^{®} retard tablets contain a prolonged release matrix of hypromellose. These tablets are in accordance with WO 03/035053 A1 and with the comparative examples described hereinafter.

### Example 1 - preparation of Tapentadol dihydrogenphosphate salt:

Three different batches of Tapentadol dihydrogenphosphate were prepared, a batch of essentially pure Tapentadol dihydrogenphosphate hemihydrate, a batch of essentially pure Tapentadol dihydrogenphosphate anhydrate, and a batch comprising a mixture of the two polymorphs (= mixed form).

### Example 1.1 - Tapentadol dihydrogenphosphate mixed form:

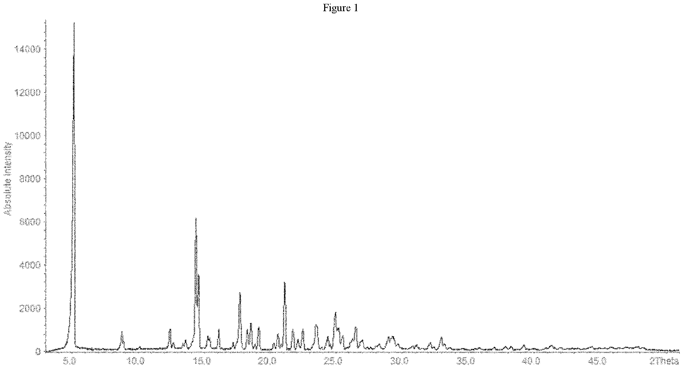

Tapentadol base (200 mg) was thoroughly mixed with phosphoric acid (104 mg, 61.7 µl, 85 wt.-%) using a spatula. The resulting mixture was aged for 4 days at 20 °C to 25 °C to give 292 mg of a white crystalline solid. The obtained product was analyzed by XRPD (Figure 1). A weight loss of 2.8% up to 172 °C was detected by TGA.

### Example 1.2 - Tapentadol dihydrogenphosphate mixed form:

Tapentadol base (7.5 g) was suspended at 20 °C to 25 °C in a mixture of ethyl acetate (81 g, 90 ml) and water (10 mg, 10 µl). The mixture was heated to 55 °C to give a clear solution. After that crystalline Tapentadol dihydrogenphosphate (10 mg) and phosphoric acid (3.9 g, 2.3 ml, 85 wt.-%) were added. Immediately a white suspension is formed. After 45 min of mixing at 60 °C the suspension was cooled to 5 °C in 45min. After further mixing at 5 °C for 45 min the resulting solid was isolated by vacuum filtration and dried (20 °C to 25 °C, vacuum, 2 h). The obtained product (9.5 g, white crystalline powder) was analyzed by XRPD. A weight loss of 3.1% up to 113 °C was detected by TGA.

### Example 1.3 Tapentadol dihydrogenphosphate mixed form:

Tapentadol base (7.5 g) was dissolved at 20 °C to 25 °C in a mixture of tetrahydrofuran (80 g, 90 ml) and water (10 mg, 10 µl). The mixture was heated to 55 °C to give a clear solution. After that crystalline Tapentadol dihydrogenphosphate (10 mg) and phosphoric acid (3.9 g, 2.3 ml, 85 wt.-%) were added. Immediately a white suspension is formed. After 45 min of mixing at 60 °C the suspension was cooled to 5 °C in 45 min. After further mixing at 5 °C for 45 min the resulting solid was isolated by vacuum filtration and dried (20 °C to 25 °C, vacuum, 2 h). The obtained product (9.6 g, white crystalline powder) was analyzed by XRPD. A weight loss of 1.9% up to 120 °C was detected by TGA.

### Example 1.4 - Tapentadol dihydrogenphosphate hemihydrate:

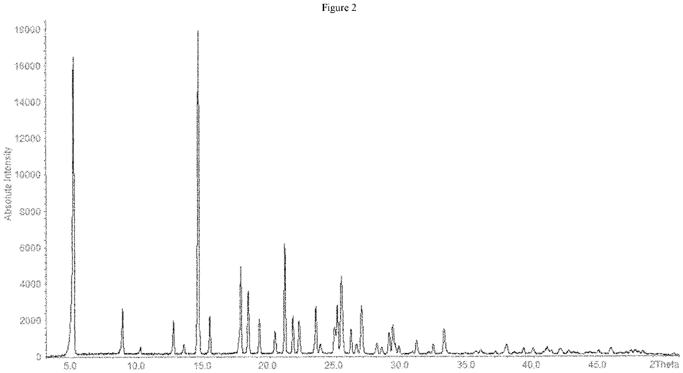

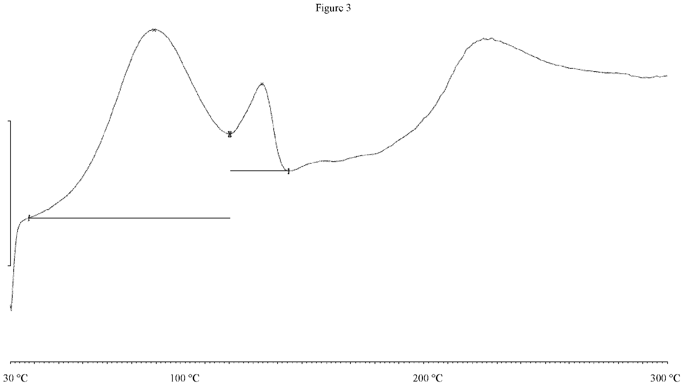

Tapentadol base (497 mg) was dissolved at 20 °C to 25 °C in a mixture of acetone (2.72 g, 3.44 ml) and water (50 mg, 50 µl) to give a clear solution. Phosphoric acid (259 mg, 153 µl, 85 wt.-%) was added and after a short time the clear solution became a suspension. The suspension was mixed for 30 min at 20 °C to 25 °C, after that cooled to 5 °C and further mixed at 5 °C for 30 min. The resulting solid was isolated by vacuum filtration and dried (20 °C to 25 °C, vacuum, 30 min). The obtained product (404 g, white crystalline powder) was analyzed by XRPD (Figure 2) and DSC (Figure 3). The first DSC peak had a normalized integral of 149.2 J·g⁻¹, with an onset temperature of 56.7 °C and a peak temperature of 89.2 °C. The second DSC peak had a normalized integral of 22.0 J·g⁻¹, with an onset temperature of 130.1 °C and a peak temperature of 133.4 °C. At about 200 °C, decomposition occurred. A weight loss of 3.7% up to 119 °C was detected by TGA.

### Example 1.5 - Tapentadol dihydrogenphosphate hemihydrate (coarse material):

Tapentadol base (60 g) was dissolved at 20 °C to 25 °C in a mixture of tetrahydrofuran (1.5 kg, 1.641) and water (82 g, 82 ml). Phosphoric acid (31.25 g, 18.5 ml, 85 wt.-%) and crystalline Tapentadol dihydrogenphosphate (5 mg) were added. During the addition the solution became a white suspension. The resulting suspension was cooled to 10 °C in 30 min. The mixture was heated to 35 °C and mixed for 30 min followed by cooling to 5 °C in 30 min. The suspension was mixed at 5 °C for 45 min. The resulting solid was isolated by vacuum filtration and dried (20 °C to 25 °C, vacuum, 2 h). The obtained product (84.6 g, white crystalline solid) was analyzed by XRPD. A weight loss of 4.5% up to 113 °C was detected by TGA.

### Example 1.6 - Tapentadol dihydrogenphosphate anhydrate:

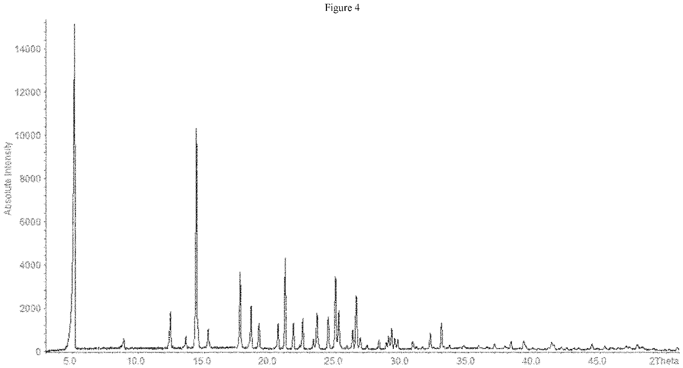

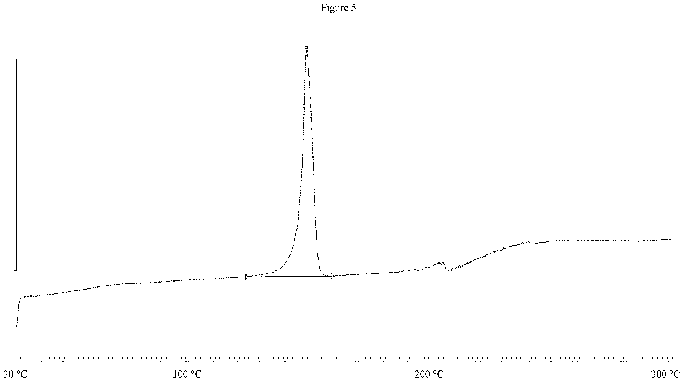

Tapentadol base (150 mg) was suspended at 20 °C to 25 °C in 1-butanol (1.2 g, 1.5 ml). The suspension was heated to 50 °C to give a clear solution. Phosphoric acid (78.1 mg, 46.3 µl, 85 wt.-%) and crystalline Tapentadol dihydrogenphosphate (5 mg) were added at 50 °C. During the addition the clear solution became a white suspension. The resulting suspension was heated to 115 °C. To keep the suspension mixable 1-butanol (400 mg, 0.5 ml) was added and mixing at 115 °C was continued for 45 min. The mixture was then cooled to 30 °C in 3 h. The resulting solid was isolated by vacuum filtration and dried (20 °C to 25 °C, vacuum, 30 min). The obtained product (157 mg, white crystalline powder) was analyzed by XRPD (Figure 4) and DSC (Figure 5). The DSC peak had a normalized integral of 72.1 J·g⁻¹, with an onset temperature of 146.5 °C and a peak temperature of 149.1 °C. At about 200 °C, decomposition occurred. A weight loss of 0.9% up to 134 °C was detected by TGA.

### Example 1.7 - Tapentadol dihydrogenphosphate anhydrate:

Tapentadol dihydrogenphosphate (500 mg, mix of hemihydrate and anhydrate) was suspended at 20 °C to 25 °C in 2-butanone (4.8 g, 6 ml) and water (5 mg, 5 µl). The reaction mixture was heated to 60 °C and mixed for 3 h. The resulting solid was isolated by hot filtration using vacuum and dried

(20 °C to 25 °C, vacuum, 30 min). The obtained product (452 mg, white crystalline powder) was analyzed by XRPD. A weight loss of 1.0% up to 129 °C was detected by TGA.

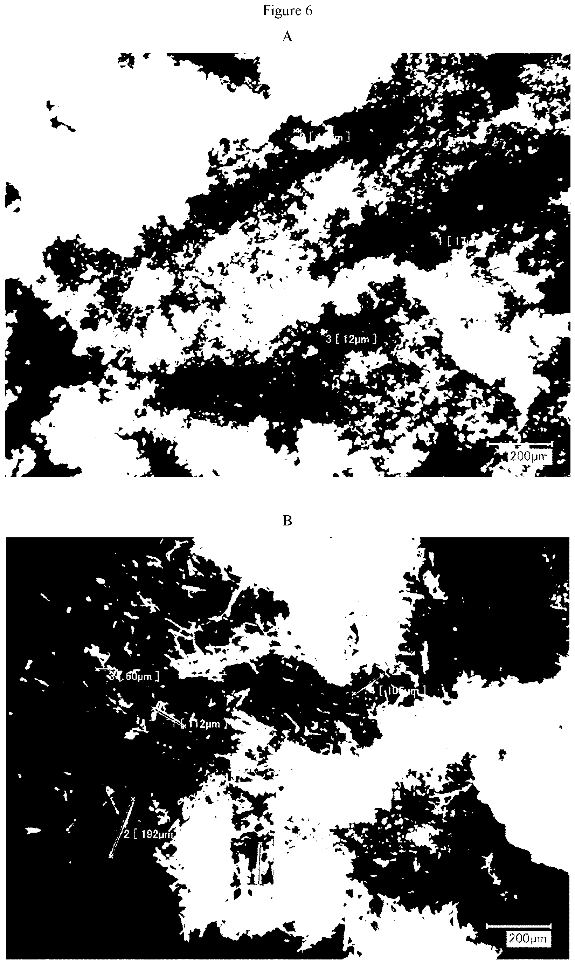

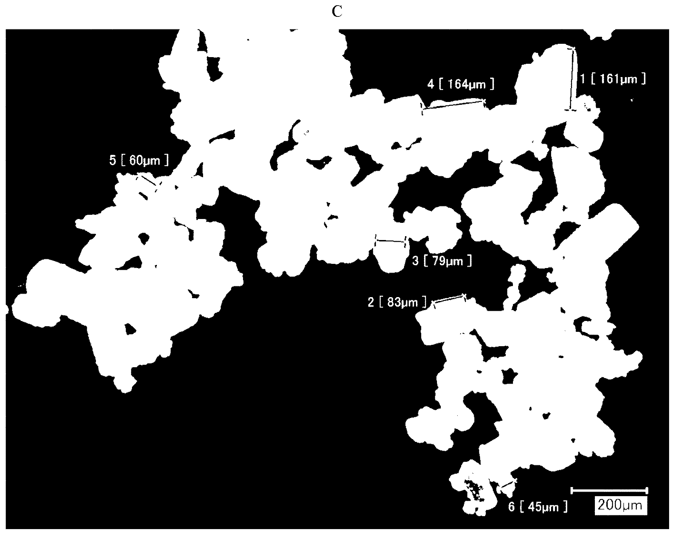

Figure 6 shows three photographs of different salts of Tapentadol that were used for manufacturing tablets. Figure 6A shows relatively fine particles of the mixed form of Tapentadol dihydrogenphosphate (average particle size upon visual inspection about 10-20 µm). Figure 6B shows relatively coarse particles of the pure hemihydrate of Tapentadol dihydrogenphosphate according to Example 1.5 (needle like crystals, average particle size upon visual inspection about 100-200 µm). Figure 6C shows relatively coarse particles of Tapentadol hydrochloride (average particle size upon visual inspection about 100-150 µm).

### Example 2 - intrinsic dissolution (dissolution rate):

100 mg of Tapentadol, either in form of the hydrochloride salt or the dihydrogenphosphate salt (mixed form [batch with fine particles], pure hemihydrate or pure anhydrate) were compressed at a compressing force of 200 kg (gravitational) for 1 minute at a surface of 0.5 cm². The thus obtained compressed samples were investigated for their dissolution rate in a Wood's apparatus at 37 °C in 900 mL of dissolution medium at different pH values and a rotational speed of the paddle of 50 rpm.

The experimental results for Tapentadol hydrochloride and for Tapentadol dihydrogenphosphate (mixed form) are compiled in the table here below:

| [%] | Tapentadol hydrochloride (comparative) | | | | | Tapentadol dihydrogenphosphate (mixed form) (inventive) | | | |
|---|---|---|---|---|---|---|---|---|---|
| min. | pH 1.0 | pH 4.5 | pH 7.4 | pH 6.8 | | pH 1.0 | pH 4.5 | pH 7.4 | pH 6.8 |
| 0 | 0 | 0 | 0 | 0 | | 0 | 0 | 0 | 0 |
| 1 | 52 | 60 | 27 | 38 | | 14 | 12 | 12 | 11 |
| 3 | 95 | 91 | 65 | 79 | | 35 | 30 | 30 | 29 |
| 5 | 99 | 95 | 80 | 92 | | 56 | 49 | 46 | 48 |
| 7 | 99 | 95 | 89 | 94 | | 78 | 73 | 63 | 66 |
| 9 | 99 | 96 | 92 | 94 | | 84 | 86 | 78 | 77 |

The experimental results for Tapentadol dihydrogenphosphate hemihydrate and for Tapentadol dihydrogenphosphate anhydrate are compiled in the table here below

| [%] | Tapentadol dihydrogenphosphate (inventive) | | | |
|---|---|---|---|---|
| | hemihydrate | | anhydrate | |
| min | pH 6.8 | pH 1.2 | pH 6.8 | pH 1.2 |
| 0 | 0 | 0 | 0 | 0 |
| 1 | 12 | 14 | 13 | 15 |
| 3 | 36 | 37 | 36 | 37 |
| 5 | 66 | 54 | 66 | 57 |
| 7 | 80 | 72 | 90 | 75 |
| 9 | 83 | 84 | 96 | 85 |

### Example 3 - thermodynamic solubility:

The thermodynamic solubility of Tapentadol hydrochloride and of Tapentadol dihydrogenphosphate (mixed form, batch with fine particles) was determined as saturation solubility in various media at various pH values. The solutions were stirred for 24 hours at 25 °C and the pH values of the solutions at the start and at the end of the experiments were measured. The dissolved amount of Tapentadol was quantified by HPLC (free base of Tapentadol). The experimental results are compiled in the table here below:

| | Tapentadol hydrochloride (comparative) | | | | Tapentadol dihydrogenphosphate (mixed form) (inventive) | | |
|---|---|---|---|---|---|---|---|
| | pH | | assay expressed as base | | pH | | assay expressed as base |
| | start | end | g/100 mL | | start | end | g/100 mL |
| 0.1 M HCl | 1.11 | 0.83 | 31.2 | | 1.11 | 2.55 | 24.3 |
| acetate pH 4.5 | 4.54 | 4.29 | 32.7 | | 4.54 | 2.81 | 21.2 |
| water | n.d. | 4.63 | 33.0 | | nd | 2.75 | 21.4 |
| citrate pH 6.8 | 6.83 | 6.17 | 31.8 | | 6.83 | 3.15 | 19.3 |
| SIF sp pH 6.8 | 6.82 | 6.23 | 32.4 | | 6.82 | 2.81 | 20.2 |
| citrate pH 7.4 | 7.39 | 6.33 | 31.9 | | 7.39 | 3.16 | 19.0 |
| 0.15N NaOH | 13.08 | 8.21 | 6.6 | | 13.08 | 8.09 | 6.5 |

| | | | | | | | |
|---|---|---|---|---|---|---|---|
| n.d. = not determined | | | | | | | |

### Example 4 - in vitro dissolution profiles of pharmaceutical dosage forms:

Tablets having the following composition were prepared by mixing all ingredients and compressing the resultant mixtures:

| | comparative | | inventive | | |
|---|---|---|---|---|---|
| | C-1 | C-2 | I-1 | I-2 | I-3 |
| [mg] | | | | | |
| Tapentadol hydrochloride¹ | 250.00 | 25.00 | - | - | - |
| fine Tapentadol dihydrogenphosphate^{1,2,3} | - | - | 250.00 | 25.00 | - |
| coarse Tapentadol dihydrogenphosphate⁴ | - | - | - | - | 250.00 |
| hydroxypropylmethylcellulose 100,000 mPa·s Typ 2208 | 100.00 | 100.00 | 100.00 | 100.00 | 100.00 |
| silicified microcrystalline cellulose (Prosolv HD90) | 304.80 | 79.79 | 304.80 | 79.79 | 304.80 |
| magnesium stearate | 4.00 | 1.09 | 4.00 | 1.09 | 4.00 |
| mean breaking strength, Ph. Eur. 2.9.8. [N] | 222 | 209 | 222 | 218 | 205 |
| mean tableting force upper punch [kN] | 12.8 | 9.3 | 7.4 | 6.3 | 8.3 |
| mean tableting force lower punch [kN] | 11.3 | 8.1 | 5.0 | 4.9 | 4.3 |

| | | | | | |
|---|---|---|---|---|---|
| ¹ equivalent dose based on the free base of Tapentadol ² mixed form ³ relatively fine particle size (average diameter about 10-20 µm upon visual inspection) ⁴ relatively coarse particle size (average diameter about 100-200 µm upon visual inspection) | | | | | |

It becomes clear from the above comparative data that for tablets of the same dose strength of Tapentadol, a significantly lower compression force is necessary when Tapentadol is employed as salt with phosphoric acid instead of hydrochloric acid in order to provide tablets of comparable breaking strength (see e.g. C-1 vs. 1-1; C-2 vs. I-2). For example, tablets C-1 and 1-1 both have a mean breaking strength of 222 N; the hydrochloride salt requires tableting forces of 12.8 kN and 11.3 kN, respectively, whereas the phosphoric acid salt only requires 7.4 kN and 5.0 kN, respectively.

Further, it becomes clear from the above comparative date that this effect does not significantly depend upon the particle size of the salt of Tapentadol with phosphoric acid (see C-1 vs. I-1 and I-3). The coarser particles of the Tapentadol salt employed in Example I-3 still require low tableting forces like the finer particles of the Tapentadol salt employed in Example I-1. In both cases, tableting forces are significantly below those of Example C-1 (Tapentadol hydrochloride).

The *in vitro* dissolution of Tapentadol from the tablets (drug load 250 mg, C-1, I-1 and I-3) was measured in a paddle apparatus in accordance with Ph. Eur., equipped with sinker, at a rotational speed of 50 rpm at 37 °C in 900 ml of different dissolution media having different pH values. The experimental results are compiled in the table here below:

| [%] | Tapentadol hydrochloride (n=3): | | | | | Tapentadol dihydrogenphosphate (n=3): | | | | |
|---|---|---|---|---|---|---|---|---|---|---|
| | C-1 | | | | | I-1 | | | | I-3 |
| time [min] | pH 6.8 | pH 4.5 | pH 1.0 | pH 1.0 40 vol.-% ethanol | | pH 6.8 | pH 4.5 | pH 1.0 | pH 1.0 40 vol.-% ethanol | pH 4.5 |
| 0 | 0 | 0 | 0 | 0 | | 0 | 0 | 0 | 0 | 0 |
| 30 | 17 | 19 | 18 | 14 | | 14 | 15 | 16 | 10 | 15 |
| 60 | 26 | 28 | 27 | 22 | | 23 | 23 | 26 | 18 | 23 |
| 90 | 33 | 36 | 34 | 28 | | 30 | 30 | 33 | 24 | 30 |
| 120 | 39 | 42 | 40 | 33 | | 35 | 36 | 39 | 29 | 36 |
| 150 | 45 | 48 | 46 | 38 | | 41 | 41 | 45 | 33 | 41 |
| 180 | 49 | 54 | 51 | 42 | | 45 | 46 | 50 | 36 | 46 |
| 210 | 54 | 58 | 55 | 46 | | 49 | 50 | 54 | 40 | 50 |
| 240 | 58 | 63 | 60 | 50 | | 53 | 54 | 58 | 43 | 54 |
| 270 | 62 | 67 | 64 | 53 | | 57 | 58 | 62 | 46 | 58 |
| 300 | 66 | 71 | 67 | 57 | | 61 | 62 | 66 | 48 | 61 |
| 330 | 69 | 74 | 71 | 60 | | 64 | 65 | 69 | 51 | 64 |
| 360 | 72 | 77 | 74 | 63 | | 67 | 68 | 72 | 53 | 67 |
| 390 | 75 | 80 | 77 | 65 | | 70 | 71 | 75 | 55 | 70 |
| 420 | 78 | 83 | 79 | 68 | | 73 | 74 | 78 | 58 | 73 |
| 450 | 80 | 85 | 82 | 70 | | 75 | 77 | 80 | 60 | 76 |
| 480 | 82 | 87 | 84 | 73 | | 78 | 79 | 83 | 62 | 78 |
| 510 | 84 | 89 | 86 | 75 | | 80 | 82 | 85 | 64 | 81 |
| 540 | 86 | 91 | 88 | 77 | | 82 | 84 | 87 | 65 | 83 |
| 570 | 88 | 92 | 89 | 79 | | 84 | 86 | 88 | 67 | 86 |
| 600 | 89 | 93 | 91 | 81 | | 86 | 88 | 90 | 69 | 88 |
| 630 | 90 | 94 | 92 | 82 | | 88 | 90 | 91 | 71 | 90 |
| 660 | 92 | 95 | 93 | 84 | | 89 | 91 | 93 | 72 | 91 |
| 690 | 93 | 96 | 94 | 85 | | 91 | 92 | 94 | 74 | 93 |
| 720 | 93 | 96 | 95 | 87 | | 92 | 93 | 95 | 76 | 94 |

### Reference Example 5 - in vitro dissolution profiles of pharmaceutical dosage forms:

Tablets having the following composition were prepared by mixing all ingredients and compressing the resultant mixtures:

| | comparative | reference |
|---|---|---|
| [mg] | C-3 | I-4 |
| Tapentadol hydrochloride¹ | 250.00 | - |
| Tapentadol dihydrogenphosphate^{1,2,3} | - | 250.00 |
| Kollidon^{®} SR | 320.00 | 320.00 |
| Aerosil 200 | 6.80 | 6.80 |
| magnesium stearate | 3.20 | 3.20 |
| mean breaking strength, Ph. Eur. 2.9.8. [N] | 237 | 230 |
| mean tableting force upper punch [kN] | 12.4 | 8.1 |
| mean tableting force lower punch [kN] | 9.9 | 4.4 |

| | | |
|---|---|---|
| ¹ equivalent dose based on the free base of Tapentadol ² mixed form ³ relatively fine particle size (average diameter about 20 µm upon visual inspection) | | |

The *in vitro* dissolution of Tapentadol from the tablets (drug load 250 mg, I-4) was measured in a paddle apparatus in accordance with Ph. Eur., equipped with sinker, at a rotational speed of 50 rpm at 37 °C in 900 ml of 0.1 N HCl without ethanol (pH 1.0) and in 0.1 N HCl with 40 vol.-% ethanol (pH 1.0). The experimental results are compiled in the table here below:

| time [min] | pH 1.0 [%] | pH 1.0 40 vol.-% ethanol [%] |
|---|---|---|
| 0 | 0 | 0 |
| 30 | 30 | 14 |
| 60 | 40 | 21 |
| 90 | 47 | 26 |
| 120 | 53 | 30 |
| 150 | 58 | 34 |
| 180 | 63 | 38 |
| 210 | 67 | 42 |
| 240 | 71 | 45 |
| 270 | 75 | 49 |
| 300 | 78 | 53 |
| 330 | 81 | 56 |
| 360 | 84 | 60 |
| 390 | 87 | 63 |
| 420 | 89 | 67 |
| 450 | 91 | 71 |
| 480 | 92 | 76 |
| 510 | 94 | 81 |
| 540 | 95 | 85 |
| 570 | 96 | 87 |
| 600 | 97 | 89 |
| 630 | 97 | 91 |
| 660 | 98 | 93 |
| 690 | 98 | 94 |
| 720 | 98 | 95 |

It becomes clear from the above comparative data that the reduced tableting force of the phosphoric acid salt of Tapentadol compared to the hydrochloride salt of Tapentadol does not depend upon the other excipients of the tablet (C-1, I-1, I-3 all hydroxypropylmethylcellulose) but is also observed with Kollidon^{®} SR. Again, for tablets of the same dose strength of Tapentadol, a significantly lower compression force is necessary when Tapentadol is employed as salt with phosphoric acid instead of hydrochloric acid in order to provide tablets of comparable breaking strength (see C-3 vs. I-4). Tablets C-3 and I-4 both have a mean breaking strength of about 235 N; the hydrochloride salt requires tableting forces of 12.4 kN and 9.9 kN, respectively, whereas the phosphoric acid salt only requires 8.1 kN and 4.4 kN, respectively.

## Claims

1. A pharmaceutical dosage form comprising Tapentadol for oral administration twice daily;
wherein Tapentadol is present as a salt with phosphoric acid, which is a crystalline form and/or amorphous form of the dihydrogenphosphate salt of Tapentadol, a solvate, an ansolvate, and/or a polymorph thereof;
wherein the weight equivalent dose of Tapentadol that is contained in the pharmaceutical dosage form is within the range of from 10 to 300 mg based on the free base of Tapentadol.
wherein the dosage form is a tablet;
wherein the dosage form provides prolonged release of Tapentadol; and
wherein Tapentadol is embedded in a prolonged release matrix comprising a prolonged release matrix material selected from hydroxypropylmethylcellulose (HPMC).

2. The pharmaceutical dosage form according to claim 1, wherein the tablet is monolithic.

3. The pharmaceutical dosage form according to any of the preceding claims, wherein the tablet has a breaking strength of at least 100 N, determined according to Ph. Eur. 2.9.8.

4. The pharmaceutical dosage form according to any of the preceding claims, wherein the weight equivalent dose of Tapentadol that is contained in the pharmaceutical dosage form amounts to 25 mg, 50 mg, or 100 mg, in each case based on the free base of Tapentadol; and wherein the dosage form has a total weight within the range of from 150 to 750 mg.

5. The pharmaceutical dosage form according to any of claims 1 to 3, wherein the weight equivalent dose of Tapentadol that is contained in the pharmaceutical dosage form amounts to 150 mg, 200 mg or 250 mg, in each case based on the free base of Tapentadol; and wherein the dosage form has a total weight within the range of from 300 to 1200 mg.

6. The pharmaceutical dosage form according to any of the preceding claims, wherein the content of the prolonged release matrix material is within the range of 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, or 65±10 wt.-%, or 70±10 wt.-%, or 75±10 wt.-%, or 80±10 wt.-%, in each case relative to the total weight of the dosage form.

7. The pharmaceutical dosage form according to any of the preceding claims for use in the treatment of pain.

8. The pharmaceutical dosage form for use according to claim 7, wherein the dosage form is orally administered.

9. The pharmaceutical dosage form for use according to claim 7 or 8, wherein the dosage form is administered twice daily.

10. The pharmaceutical dosage form for use according to any of claims 7 to 9, which after oral administration provides plasma levels of Tapentadol providing pain relief for a duration of at least 6 hours.

11. The pharmaceutical dosage form for use according to any of claims 7 to 10, wherein the pain is chronic pain.

12. A method for the preparation of a pharmaceutical dosage form according to any of claims 1 to 11, the method comprising the steps of
(a) providing a mixture containing essentially the total amount of Tapentadol to be contained in the dosage form and at least one prolonged release matrix material selected from hydroxypropylmethylcellulose (HPMC), optionally together with one or more excipients;
(b) optionally granulating the mixture provided in step (a) thereby obtaining a granulate;
(c) optionally mixing the granulate obtained in step (b) with one or more excipients thereby obtaining a granulate mixture;
(d) compressing the mixture provided in step (a) or the granulate obtained in step (b) or the granulate mixture obtained in step (c) into tablets;
(e) optionally, film coating the tablets compressed in step (d).

13. The method according to claim 12, wherein compressing in step (d) is performed at a compression force of not more than 20 kN, more preferably not more than 15 kN, still more preferably not more than 10 kN, yet more preferably not more than 9.5 kN, even more preferably not more than 9.0 kN, most preferably not more than 8.75 kN, and in particular not more than 8.5 kN.

## Patentansprüche

1. Tapentadol umfassende pharmazeutische Dosierungsform für die zweimal tägliche Verabreichung;
wobei Tapentadol als Salz mit Phosphorsäure vorliegt, die eine kristalline Form und/oder amorphe Form des Dihydrogenphosphatsalzes von Tapentadol, ein Solvat, ein Ansolvat und/oder ein Polymorph davon ist;
wobei die Gewichtsäquivalentdosis von Tapentadol, die in der pharmazeutischen Dosierungsform enthalten ist, im Bereich von 10 bis 300 mg, bezogen auf die freie Base von Tapentadol, liegt; wobei die Dosierungsform eine Tablette ist;
wobei die Dosierungsform eine verlängerte Freisetzung von Tapentadol bereitstellt; und
wobei Tapentadol in einer Matrix mit verlängerter Freisetzung eingebettet ist, die ein Matrixmaterial mit verlängerter Freisetzung umfasst, das aus Hydroxypropylmethylcellulose (HPMC) ausgewählt ist.

2. Pharmazeutische Dosierungsform nach Anspruch 1, wobei die Tablette monolithisch ist.

3. Pharmazeutische Dosierungsform nach einem der vorhergehenden Ansprüche, wobei die Tablette eine Bruchfestigkeit von mindestens 100 N aufweist, bestimmt gemäß Ph. Eur. 2.9.8.

4. Pharmazeutische Dosierungsform nach einem der vorhergehenden Ansprüche, wobei die gewichtsäquivalente Dosis von in der pharmazeutischen Dosierungsform enthaltenem Tapentadol 25 mg, 50 mg oder 100 mg, jeweils bezogen auf die freie Base von Tapentadol beträgt; und wobei die Dosierungsform ein Gesamtgewicht im Bereich von 150 bis 750 mg hat.

5. Pharmazeutische Dosierungsform nach einem der Ansprüche 1 bis 3, wobei die gewichtsäquivalente Dosis von in der pharmazeutischen Dosierungsform enthaltenem Tapentadol 150 mg, 200 mg oder 250 mg, jeweils bezogen auf die freie Base von Tapentadol beträgt; und wobei die Dosierungsform ein Gesamtgewicht im Bereich von 300 bis 1.200 mg hat.

6. Pharmazeutische Dosierungsform nach einem der vorhergehenden Ansprüche, wobei der Gehalt an Matrixmaterial mit verlängerter Freisetzung vorzugsweise im Bereich von 15±10 Gew.-% oder 20±10 Gew.-% oder 25±10 Gew.-% oder 30±10 Gew.-% oder 35±10 Gew.-% oder 40±10 Gew.-% oder 45±10 Gew.-% oder 50110 Gew.-% oder 55±10 Gew.-% oder 60±10 Gew.-% oder 65±10 Gew.-% oder 70±10 Gew.-% oder 75±10 Gew.-% oder 80±10 Gew.-%, jeweils bezogen auf das Gesamtgewicht der Dosierungsform, liegt.

7. Pharmazeutische Dosierungsform nach einem der vorhergehenden Ansprüche zur Verwendung bei der Behandlung von Schmerzen.

8. Pharmazeutische Dosierungsform zur Verwendung nach Anspruch 7, wobei die Dosierungsform oral verabreicht wird.

9. Pharmazeutische Dosierungsform zur Verwendung nach Anspruch 7 oder 8, wobei die Dosierungsform zweimal täglich verabreicht wird.

10. Pharmazeutische Dosierungsform zur Verwendung nach einem der Ansprüche 7 bis 9, welche nach oraler Verabreichung Plasmaspiegel von Tapentadol bereitstellt, die über eine Dauer von mindestens 6 Stunden Schmerzlinderung bereitstellen.

11. Pharmazeutische Dosierungsform zur Verwendung nach einem der Ansprüche 7 bis 10, wobei es sich bei den Schmerzen um chronische Schmerzen handelt.

12. Verfahren zur Herstellung einer pharmazeutischen Dosierungsform nach einem der Ansprüche 1 bis 11, wobei das Verfahren die folgenden Schritte umfasst:
(a) das Bereitstellen einer im Wesentlichen die gesamte Menge an Tapentadol, die in der Dosierungsform enthalten sein soll, und mindestens ein Matrixmaterial mit verlängerter Freisetzung, das aus Hydroxypropylmethylcellulose (HPMC) ausgewählt ist, enthaltenden Mischung, gegebenenfalls zusammen mit einem oder mehreren Exzipienten;
(b) gegebenenfalls das Granulieren der in Schritt (a) bereitgestellten Mischung unter Erhalt eines Granulats:
(c) gegebenenfalls das Mischen des in Schritt (b) erhaltenen Granulats mit einem oder mehreren Exzipienten unter Erhalt einer Granulatmischung;
(d) das Verpressen der in Schritt (a) bereitgestellten Mischung oder des in Schritt (b) erhaltenen Granulats oder der in Schritt (c) erhaltenen Granulatmischung zu Tabletten;
(e) gegebenenfalls das Filmbeschichten der in Schritt (d) verpressten Tabletten.

13. Verfahren nach Anspruch 12, wobei das Verpressen in Schritt (d) mit einer Kompressionskraft von nicht mehr als 20 kN, besonders bevorzugt nicht mehr als 15 kN, noch mehr bevorzugt nicht mehr als 10 kN, noch mehr bevorzugt nicht mehr als 9,5 kN, noch mehr bevorzugt nicht mehr als 9,0 kN, am meisten bevorzugt nicht mehr als 8,75 kN und insbesondere nicht mehr als 8,5 kN erfolgt.

## Revendications

1. Forme posologique pharmaceutique comprenant du Tapentadol pour une administration orale deux fois par jour ;
dans laquelle le Tapentadol est présent sous forme de sel avec l'acide phosphorique, qui est une forme cristalline et/ou une forme amorphe du sel dihydrogénophosphate de Tapentadol, un solvate, un ansolvate et/ou un polymorphe de celui-ci ;
dans laquelle la dose équivalente en poids de Tapentadol qui est contenue dans la forme posologique pharmaceutique se situe dans la plage de 10 à 300 mg sur la base de la base libre de Tapentadol.
dans laquelle la forme posologique est un comprimé ;
dans laquelle la forme posologique fournit une libération prolongée de Tapentadol ; et
dans laquelle le Tapentadol est intégré dans une matrice à libération prolongée comprenant un matériau de matrice à libération prolongée choisi parmi l'hydroxypropylméthylcellulose (HPMC).

2. Forme posologique pharmaceutique selon la revendication 1, dans laquelle le comprimé est monolithique.

3. Forme posologique pharmaceutique selon l'une quelconque des revendications précédentes, dans laquelle le comprimé a une résistance à la rupture d'au moins 100 N, déterminée selon Ph. Eur. 2.9.8.

4. Forme posologique pharmaceutique selon l'une quelconque des revendications précédentes, dans laquelle la dose équivalente en poids de Tapentadol qui est contenue dans la forme posologique pharmaceutique s'élève à 25 mg, 50 mg ou 100 mg, dans chaque cas sur la base de la base libre de Tapentadol ; et dans laquelle la forme posologique a un poids total dans la plage de 150 à 750 mg.

5. Forme posologique pharmaceutique selon l'une quelconque des revendications 1 à 3, dans laquelle la dose équivalente en poids de Tapentadol qui est contenue dans la forme posologique pharmaceutique s'élève à 150 mg, 200 mg ou 250 mg, dans chaque cas sur la base de la base libre de Tapentadol ; et dans laquelle la forme posologique a un poids total dans la plage de 300 à 1 200 mg.

6. Forme posologique pharmaceutique selon l'une quelconque des revendications précédentes, dans laquelle la teneur en matériau de matrice à libération prolongée est dans la plage de 15 ± 10 % en poids, ou 20 ± 10 % en poids, ou 25 ± 10 % en poids, ou 30 ± 10 % en poids, ou 35 ± 10 % en poids, ou 40 ± 10 % en poids, ou 45 ± 10 % en poids, ou 50 ± 10 % en poids, ou 55 ± 10 % en poids, ou 60 ± 10 % en poids, ou 65 ± 10 % en poids, ou 70 ± 10 % en poids, ou 75 ± 10 % en poids, ou 80 ± 10 % en poids, dans chaque cas par rapport au poids total de la forme posologique.

7. Forme posologique pharmaceutique selon l'une quelconque des revendications précédentes destinée à être utilisée dans le traitement de la douleur.

8. Forme posologique pharmaceutique destinée à être utilisée selon la revendication 7, dans laquelle la forme posologique est administrée par voie orale.

9. Forme posologique pharmaceutique destinée à être utilisée selon la revendication 7 ou 8, dans laquelle la forme posologique est administrée deux fois par jour.

10. Forme posologique pharmaceutique destinée à être utilisée selon l'une quelconque des revendications 7 à 9, qui, après administration orale, fournit des taux plasmatiques de Tapentadol procurant un soulagement de la douleur pendant une durée d'au moins 6 heures.

11. Forme posologique pharmaceutique destinée à être utilisée selon l'une quelconque des revendications 7 à 10, dans laquelle la douleur est une douleur chronique.

12. Procédé de préparation d'une forme posologique pharmaceutique selon l'une quelconque des revendications 1 à 11, le procédé comprenant les étapes
(a) de fourniture d'un mélange contenant essentiellement la quantité totale de Tapentadol devant être contenue dans la forme posologique et au moins un matériau de matrice à libération prolongée choisi parmi l'hydroxypropylméthylcellulose (HPMC), éventuellement avec un ou plusieurs excipients ;
(b) éventuellement de granulation du mélange fourni à l'étape (a) pour obtenir ainsi un granulat ;
(c) éventuellement de mélange du granulat obtenu à l'étape (b) avec un ou plusieurs excipients pour obtenir ainsi un mélange de granulats ;
(d) de compression du mélange fourni à l'étape (a) ou du granulat obtenu à l'étape (b) ou du mélange de granulats obtenu à l'étape (c) en comprimés ;
(e) éventuellement, d'enrobage avec un film des comprimés compressés à l'étape (d).

13. Procédé selon la revendication 12, dans lequel la compression à l'étape (d) est effectuée à une force de compression non supérieure à 20 kN, plus préférablement non supérieure à 15 kN, encore plus préférablement non supérieure à 10 kN, davantage plus préférablement non supérieure à 9,5 kN, plus préférablement encore non supérieure à 9,0 kN, le plus préférablement non supérieure à 8,75 kN, et en particulier non supérieure à 8,5 kN.
